Europäisches Patentamt

(19) European Patent Office

Office européen des brevets

(11) **EP 0 470 596 B1**

(12) **EUROPEAN PATENT SPECIFICATION**

(45) Date of publication and mention
of the grant of the patent:
**17.01.1996 Bulletin 1996/03**

(51) Int. Cl.⁶: **A61L 15/40**

(21) Application number: **91113273.6**

(22) Date of filing: **07.08.1991**

(54) **An absorbent pad, a method of obtaining an absorbent pad, and a disposable absorbent article**

Saugkissen, Verfahren zur Herstellung eines Saugkissens und absorbierender Einwegartikel

Tampon absorbant, méthode pour la fabrication d'un tampon absorbant et article absorbant jetable

(84) Designated Contracting States:
**DE ES FR GB IT**

(30) Priority: **08.08.1990 BR 9003886**

(43) Date of publication of application:
**12.02.1992 Bulletin 1992/07**

(73) Proprietor: **McNEIL-PPC, INC.**
**Milltown New Jersey 08850 (US)**

(72) Inventor: **Kotsevitis,Marcia Leandro**
**Sao Jose dos Campos Sao Paulo (BR)**

(74) Representative: **Strehl Schübel-Hopf Groening &**
**Partner**
**D-80538 München (DE)**

(56) References cited:
**EP-A- 0 063 331**          **FR-A- 2 598 280**

**Description**

The present invention refers to an absorbent pad of vegetable origin, suitable for use in disposable absorbent articles.

Disposable absorbent articles, such as diapers, women's hygienic pads and compresses, usually contain absorbent layers of woodpulp fibers. This pulp is derived from trees that take from 12 to 20 years to reach the utilization state.

Thus, in order to supply the demand for woodpulp for use in disposable absorbent articles, large areas are required for the plantation of trees such as eucalypts, pine tries and others. Although woodpulp is the most economical material for use in disposable articles at present, there is a demand for alternatives that are easier and cheaper to utilize, preferably from renewable sources.

Thus, researches have been directed to the recycling of wastes in general as alternative sources of cheap raw materials, especially agricultural residues.

As a result of the search for alternative absorbent materials for use in disposable articles, the following patents or patent applications have been proposed: US 4737582, EP 137608 and EP 137611 among others, which mention the utilization of agricultural residues having pectin contents higher than 15% and derived from, for instance, orange peel, apple peel, watermelon peel, beetroot residues and others.

Several other patents referring to absorbent materials derived from agricultural sources can be mentioned, for example US 4676871, US 4618496, US 4473440, and CA 1167678, which relate to the utilization of turf for this purpose.

On the other hand, as regards the culture of maize, it is known that the it becomes ripe for harvest five or six months after being planted, and one of the indications that it is ready for harvest is when the stem is dry. After the ears are harvested, the stalk is of no use at all for later harvest and is usually broken and left on the soil, where it may serve as organic manure. There are cases in which, for instance, the plantation area is chemically fertilized; the dry stalks are removed, burned or used as food complement for cattle, for lack of a better utilization.

However, no relevant commercial use is known for the maize stems after the harvest. The prior art discloses, however, many possible ways of using the maize stem as a lignocellulosic raw material, as can be seen, for instance, from the following patents:

- US 4644060, which refers to the obtention of a substrate rich in monosaccharides available for alcohol obtention fermentation, after reaction with ammonia,
- US 4814944, which refers to the obtention of a complement for wheat, for instance, in making bread, by reaction with alkaline peroxide,
- US 4566380 and US 4649113, which refer to the obtention of animal food or a nutrient for microorganisms in fermentation, by reaction with alkaline peroxide,
- US 4738815 and US 4605640, which refer to the obtention of an oil absorbent material from oil emulsions in water after reaction with quaternary fatty ammonia salts.

Other uses for the maize stem are cited in the following patents:

- DE 2151326, a substrate for growing wood-destroying fungus,
- US 4519340, which refers to a component in a mixture with sawdust and soda for absorbing animal excrement, placed in stables, which provides accelerated degradation in mixture with earth.

The present invention, however, is based on the verification that the maize stem can be effectively used as an absorbent in absorbent pads and articles, through a simple and inexpensive processing, thus providing another alternative source for the production of absorbents.

Therefore, the objective of the present invention is to provide an absorbent pad useful for disposable absorbent articles derived from maize stem, as well as for its obtention, in which the granulated material is structured in a specific manner.

A further objective of the present invention is the obtention of disposable absorbent articles, for example, diapers, women's hygienic pads and compresses, which contain a granulated material and/or an absorbent pad derived from maize stem.

According to the present invention, a granulated material is disclosed, which is obtained from maize stem and has important absorbent properties, suitable for use in disposable absorbent articles through a simple and inexpensive processing.

The raw material, namely maize stem, is abundant, is planted in an intensive manner in innumerable regions of the world, has a low cost - since its stem is often thrown away after the annual harvest - and has a rapid renewal cycle as a function of the annual harvest.

Anyway, the raw material of the absorbent of the invention is inexpensive, since it is a plant residue, regularly available in large quantities in the world, for it is planted every year.

A conservative estimate shows that, by using only the pith of the maize stem and eliminating the husk and nodes, one hectare of ground cultivated with maize supplies 400 kg of raw material as preferred for the invention, that is to say, more than 12 million tons per harvest in the United States alone.

Maize stem is changed into an absorbent material, after conversion into a material having an adequate granulometry. This material can be either utilized as such or advantageously changed into an absorbent pad structured with fibers, binders, sheets and the like, shaped and optionally flexibilized to conform to utilization as disposable absorbent articles.

One of the advantages of the invention is that the absorbing capacity of the granulated material - although varying with the origin of the maize stem, with the granulometry and with the treatment given to the material - can be equal or superior to that of woodpulp, thus creating the opportunity of reducing the apparent volume of the disposable absorbent articles that containing same for an equivalent holding capacity. Another advantage of the material of the invention over woodpulp is its greater capacity of holding the liquid absorbed when subjected to pressure by virtue of the cellular structure of the pith of the maize stem.

As used herein, "granulated form" means a particulate state composed by grains, granules, particles, slices and the like, without any indication of dimension, shape or homogeneity, indicating only the fragmentation of the maize stem.

Said absorbent material preferably comprises the pith fraction of the maize stem without the husk (outer coating) and the nodes (the cross sections which are harder than the rest, spaced regularly along the stem).

The granulometry of the material can be any one, and it is up to those skilled in the art to choose the one that conforms to the desired purpose better.

In a particular embodiment of the invention, the granulometry of the material comprises a continuous range, for instance, containing from the fine powder to grains retained in a 1.68mm (10 mesh) sieve.

Advantageously the equivalent to the granulometric fraction comprised between 1.68 mm (10)and 0.105 mm (150 mesh), preferably between 1.68 mm (10) and 0.149 mm (100 mesh), and more preferably between 1.68 mm (10) and 0.250 mm (60 mesh) is utilized.

In this selection of granulometry of the new absorbent material, one should take into account that bigger grains are more absorbent, less densely compacted and easier to handle than finer grains, which are less absorbent and more difficult to handle, but have a more rapid absorption and provide a more rapid spreading of liquid.

Optionally, the granulated material of the invention can be bleached in view of the utilization in which this aspect is desired.

The present invention also refers to the method of obtaining a granulated absorbent material derived from maize stems, in which said stems are subjected to a fragmentation process.

Preferably the fragmentation is carried out by mechanical means such as grinding, milling or pressing, which do not substantially destroy the cellular structure of the pith of the maize stem.

In an advantageous manner, a wet-grinding is carried out, in which the material to be fragmented is immersed into water, ground, and then drained and dried.

Also in an advantageous manner, the grinding is preceded by husking and denoding the stem in order to remove these parts that are less absorbent than the pith.

After fragmentation the material can be granulometrically separated by any known means in the fraction comprised between 1.68 mm (10) and 0.105 mm (150 mesh), preferably between 1.68 mm (10) and 0.149 mm (100 mesh)and more preferably between 1.68mm (10) and 0.250 mm (60 mesh).

Still optionally, the granulated material can be subjected to a treatment that intensifies its absorbption power. For instance, a bath with an aqueous surfactant solution will increase the surface energy of the material of the invention and bring about a greater wettability. In a particular manner, this treatment is carried out during the wet-grinding of the maize stem, when a surfactant is placed into the water of the grinding container.

If derised, the granulated material of the invention can be treated also in a manner known in the art, to avoid attacks by microorganisms that cause degradation of both the physical properties and the aspect of the material.

Another feature of this invention refers to an absorbent pad, characterized by comprising a structured granulated material derived from maize stem.

As used here, "pad" means a substantially joined body as compared with an amount of loose granulated material. In this regard, the body is sufficiently joined to be handled or carried without disjoining substantially.

As used here, "structured" refers to the presence of chemical or mechanical structuring means that provide cohesion among the grains of the granulated material.

The pad of the invention can comprise, as structuring means, one or more structuring materials in contact with the granulated material derived from maize stem, namely:

## (A) BINDERS

- any product or material that provides adhesion of the grains among themselves. Its nature or composition varies depending upon the kind of application on the granulated material. For instance, if the granulated material is subjected to immersion in an aqueous binder bath (to be drained and dried later, for the purpose of obtaining the adhesion of the grains among themselves), the binder is a material having characteristics that allow its solution or dispersion in water. A few non-limitative examples are given below:

- polymers or copolymers dispersions of polyvinylic alcohol, vinyl acetate, acrylic acid and their esters, styrene and others;
- solutions of gum arabic, xantan gum, guar gum, pectin, modified cellulose and others.

Thus, the binder that appears as a component of the absorbent of the invention can be any one among those that can be applied by processes compatible with the physical shape of the granulated material derived from maize stem, for example:

- immersion process
- hot-melt application process
- powder application process
- spray application process
- flock application process
- fusible polymer blow application process

and that do not substantially affect the absorpotion properties of the obtained product.

(B) **FIBERS OR FILIFORM MATERIALS**

- introduced in the granulated material of the invention either randomly or not, which provide cohesion among the grains.

Such filiform materials can be of any nature, either vegetable or synthetic. Advantageously textile fibers are utilized. Long fibers give more structural stability to the pad, but are more subject to entanglement during the process of introduction. Preferably fibers chosen from polyester, polypropylene and rayon are utilized.

The more adequate fiber lengths are in the range 1 - 25 mm, preferably 2 - 15 mm and more preferably 5 - 10 mm.

In a particular manner, the fibers utilized in the invention have a tex (denier) between 0.011 (0.1) and 2.22 (20), preferably between 0.055 (0.5) and 1.11 (10) and more preferably between 0.11 (1) and 0.56 (5).

In an advantageous embodiment of the invention, the absorbent pad is structured by portions of polyester or polypropylene fibers having a tex (denier) of about 0.22 (2) and an average length of about 5mm, disposed in layers alternating with the granulated material in the fiber granulated ratio of 1:15 by weight.

(C) **SHEETS OR FOLIATE MATERIAL**

- either porous or perforated, thus allowing a substantial perviousness to liquids; they serve also as a structural support for the granulated material in both the surfaces and as intermediate layers of the absorbent pad of the invention. They can be of any nature, for instance, paper, fabric, nonwoven fabric, films or plastic webs, or any other material or form that do not substantially damage the absorption properties of the obtained pad.

Such sheets can perform an additional function of preventing the absorbed material from being apparent, which is a useful characteristic, for instance, if the absorbent pad is used as a catamenial absorbent, in which it is of interest to obliterate the vision of the absorbed blood, or further to prevent vision of the non-bleached material.

In a particular manner, sheets of paper weighing from 10 to 50 $g/m^2$, preferably from 15 to 40 $g/m^2$ and more preferably from 20 to 30 $g/m^2$, disposed on the two faces of the pad (it is understood that the pad has a very little thickness with respect to the other dimensions, so as to configure two outer and opposed surfaces).

The absorbent pad of the invention can embrace a large range of thickness or density, depending upon its obtention process and the desired purpose. In addition to providing variation of flexibility, these parameters also affect the absorption power and velocity.

Absorbent pads utilizable in disposable articles advantageously have a thickness of from 0.05 to 1.00 cm, preferably from 0.1 to 0.5 cm and more preferably from 0.15 to 0.25 cm.

Adequate apparent densities are from 0.01 to 0.7 $g/cm^3$, preferably from 0.1 to 0.5 $g/cm^3$ and more preferably from 0.3 to 0.4 $g/cm^3$.

Prererably, the absorbent pad of the invention is made up of layers of granulated material with layers of fibers inserted between them, enclosed between two sheet of paper, with a total thickness of 0.15 cm and an apparent density of 0.35 $g/cm^3$.

According to another embodiment, the present invention relates to a method of obtaining an absorbent pad by introducing one or more structuring materials in the granulated material derived from maize stem, optionally followed by a shaping operation.

As used here, the expression "by introducing structuring materials" means placing the granulated material in contact with one or more structuring materials by any known means.

If the structuring material is a fiber, the introduction operation is, for instance, the mechanical mixing of fibers and granulated material, either randomly or in alternating layers.

If, on the other hand, the structuring material is made up of sheets, the introduction operation will be, for instance, the arrangement of the granulated material in layers on the sheets in the desired amounts.

Further, if the structuring material is, for example, a molten polymer, the introduction operation can be a "melt blown" process, in which, while a molten polymer curtain comes out of and down from a melt block, the granulated material is blown or thrown against said curtain.

The operations of introducing structuring materials in accordance with the invention are dependent upon the nature of the material and obeys principles known in the chemical engineering - see, for instance, Chemical Engineers Handbook, Perry J.E., Editor, McGraw-Hill Book Co., New York, 4th edition, 1963.

"Shaping operation" is the one composed by one or more phases, in which the granulated material, already contacted with the structuring material, is shaped into a substantially joined pad. Such an operation may not be necessary when the operation of introducing structuring materials itself performs the shaping function.

For example, for a specific amount of granulated material mixed with fibers placed between two sheets, the shaping operation may be effected by hot pressing until an essentially united pad is obtained.

Another example of shaping: for an amount of granulated material contacted with a vinyl acetate/dibutyl maleate copolymer binder through an aqueous dispersion, the adequate shaping operation can be the draining of the liquid until a cake is obtained, followed by a first pressing at room temperature to remove the excess liquid and finally a second hot-pressing until the cohesion of the grains among themselves is achieved.

When fibers or sheets are utilized as structuring materials, preferably a previous wetting of the granulated material is carried out, for instance, with water spray, which improves the contact between the granulated material and the structuring material.

In the case of the granulated material blown onto a molten polymer curtain coming out of a melt block, one can achieve a substantially joined absorbent pad only with this operation, and a later shaping operation can be dispensed with.

When the granulated material is not previously treated either to increase its compatibility with liquids or to avoid attacks by microorganisms, such a treatment can be optionally effected either during or after the process of obtaining the absorbent pad. As an example of this case, the ready pad can be sprayed with an aqueous solution of anionic surfactant and/or with a bacteriostatic or bactericide product, followed by drying. The treatment delt with in this paragraph can be carried out during any phase of the method of obtaining the pad in which this is physically possible and that does not substantially affect the absorption properties of the pad.

Another embodiment of the method of obtaining the absorbent pad, according to the invention, includes a complementary flexibilization phase. The pad obtained after shaping may have a rigidity considered high for the purpose for which it is inteded. Thus, it is advantageous to subject the pad to an operation that either eliminates or reduces this rigidity down to adequate levels. This can be achieved in several ways, for instance:

- macerating the pad in a grooved calender either transversely or longitudinally, either by points or by any ageometry suitable for this purpose,
- crimping the pad, in which process, as far as a given depth, it is subjected to little ruts or grooves so as to weaken the naterial at those specific points,
- grooves or cuts with a controlled depth made in the pad - these cases, it is well for the pad to have been made with fibers in order to avoid the separation of pieces of the pad between the cuts.

The invention further refers to disposable absorbent pads comprising at least one structured absorbent pad containing granulated material derived from maize stems and/or the granulated material itself.

Disposable absorbent articles are those intende for absorbing body fluids - diapers, women's hygienic pads, compresses and the like - and then disposed without further or repeated use.

A particular embodiment of the invention in this sense is a disposable diaper comprising a liquid-pervious front sheet in contact with the skin, a liquid-impervious back sheet and, arranged between these two sheets, an amount of bleached woodpulp containing dispersed granulated material derived from maize stem previously treated with a surfactant or a biocide.

Another particular embodiment of the invention in this sense is a woman's hygienic pad comprising a perforated plastic film in contact with the user's skin, an impervious plastic back film and, arranged between these two films, a structured absorbent pad made up of: a granulated material derived from maize stem, bleached, treated with a surfactant or a biocide, alternating with layers of short polypropylene fibers, pressed between two sheets of tissue paper and macerated in a gear-type calender.

Disposable articles containing any type of materials, shapes or structure comprising at least one absorbent pad derived from the granulated material derived from maize stem and/or the granulated material itself can be obtained according to the method of the invention.

Examples of the practical realization of the present invention are given below, by way of illustration.

The absorbent pad of the invention (samples A and B) with an apparent density of 0.35 g/cm$^3$ is suitable for utilization in disposable absorbent articles. However, the cellulose pulp used commercially as an absorbent material has an apparent density of about 0.1 g/cm$^3$. For a more complete comparison of the performance between the invention and the prior art, samples of cellulose pulp having an apparent density of both 0.1 g/cm$^3$ (sample D) and 0.35 g/cm$^3$ (sample C) have been analysed.

As regards the absorbent pad of the invention, a sample (sample B) is also made in which the granulated material has been previously treated with an aqueous surfactant solution in order to show that the absorption properties obtained can be significantly heightened with a simple treatment.

According to these comments, four samples called A, B, C and D have been prepared for the purpose of comparing the performance of products of the invention and products of the prior art, namely:

- Sample A: a structured absorbent pad made up of a granulated material derived from maize stem, prepared in accordance with steps I, III and IV described below (therefore, without surfactant treatment).
- Sample B: a structured absorbent pad made up of a granulated material derived from maize stem, prepared in accordance with steps I, II, III and IV described below (therefore, with surfactant treatment).

Samples A and B are obtained with an apparent density of about 0.35 g/cm$^3$.

- Sample C: an absorbent pad having long fibers of bleached cellulose pulp obtained in accordance with steps III and IV described below.

Sample C is obtained with an apparent density of about 0.35 g/cm$^3$.

- Sample D: an absorbent pad of long fibers of bleached cellulose pulp usually employed as an absorbent means for disposable articles such as diapers and hygienic pads.

Sample D has an apparent density of 0.1 g/cm$^3$.

## STEPS IN THE METHOD OF OBTAINING SAMPLES

### I- Granulation

Maize stems (XL560 hibrid of the company Braskalb) are husked, denoded and subjected to grinding in a household Walita liquefier for 2 minutes.

Then the ground material is subjected to sieving in a Produtest vibrator (vibration at level 8 during 30 minutes) and the fraction retained between 0.168mm (10) and 0.250 mm (60 mesh) is withdrawn.

### II- Treatment with a Surfactant

20g of a granulated material from step I are placed in 1 litre of a solution with 1% of Aerosol OT 75 surfactant manufactured by American Cyanamid Co. It is stirred for 30 minutes.

Then a draining step is carried out in a Bucher funnel and drying in a drying oven with forced ventilation with air at 105°C for two hours or until the material is dry to touch.

### III- Shaping of the Pad

In this step a sheet shaper of the Kothen-Rapide type (manufactured by REGMED), composed by a shaping column and a drying press, is utilized.

16.9g of absorbent material (in the case of Sample C, this is a cellulose pulp; in the case of Sample either A or B, it is 15.9 g of a granulated material derived from maize stem plus 1g of polyester fibers having 0.24 tex (a denier of 2.2) and an average length of 5mm) are added to the shaping column under stirring caused by air injected through its base, until a visual homogeneity is achieved.

A rapid draining is made under suction until the material is completely deposited on the base web of the column in the form of a cake.

The cake obtained from homogenized material is carefully placed between two sheets of tissue paper (weighing 26 g/cm$^2$) and placed in the drying press at 90°C for 60 minutes.

## IV- Flexibilization

The absorbent pad obtained in step III is passed twice in a gear calender (diameter as far as the base of the teeth of 4.2cm, each cylinder having 54 2.5cm teeth and the cylinder being 22cm long), so that, the pad remaining grooved in a given direction after the first passage, the second passage is carried out transversely with respect to the grooves present.

## TEST METHODS

Samples A, B, C and D have been appraised for:

- liquid-absorbing capacity
- liquid-holding capacity
- draining power
- longitudinal spreading of liquids, in accordance with the methods described below.

## ABSORBING CAPACITY

For this test the GATS (Gravimetric Absorbency Testing Systems) apparatus is used, which is manufactured by M/K Systems, Massachussets, USA.

A sample of a pad of about 1g to be tested (a circle of approximately 5cm in diameter) is placed at the point source under a pressure of 3.51 g/cm$^2$ (0.05 psi).

The test begins when the liquid (an aqueous NaCl 1% solution) begins to flow freely out of the reservoir towards the sample. The equipment records the evolution of the amount of liquid sbsorbed as time passes.

The value of the absorbing capacity expressed in gram of liquid per gram of absorbent material is that achieved when the sample reaches the saturation state and does not absorb anything more.

## LIQUID-HOLDING CAPACITY

Here also the GATS apparatus mentioned in the test for absorbing capacity is used by utilizing the porous plate and a cyclic variation of pressure.

On the porous plate, maintained saturated with an aqueous NaCl 1% solution through connection with the reservoir of the apparatus, the same amount of sample of the previous test is placed and subjected to three cycles with the following pressures:

1- 3.51 g/cm$^2$ (0.05 psi) for 30 minutes
2- 35.15 g/cm$^2$ (0.5 psi) for 20 minutes
3- 3.51 g/cm$^2$ (0.05 psi) for 10 minutes.

The value of the liquid-holding capacity expressed in percentage corresponds to the amount of liquid held by Sample No. 2 with respect to that of the 3rd cycle.

The results of the tests cited above are shown in Table 1 below.

## DRAINING POWER

A pad of bleached woodpulp (4cm x 4cm, weighing about 260 g/cm$^2$, with an apparent density of 0.1 g/cm$^3$) is centralized on a sample of the absorbing material to be tested (5cm x 15cm) and remains in contact therewith. It receives three discharges of NaCl 1% solution (in zero, thirty and sixty minutes, equivalent to 7.5 ml/g of sample).

Every 5 minutes during 80 minutes, the draining is determined according to the following equation (weight is expressed in grams):

$$\text{drain}(\%) = \frac{\text{(weight of wet sample-weight - dry sample)}}{\text{weight of added liquid}} \cdot 100$$

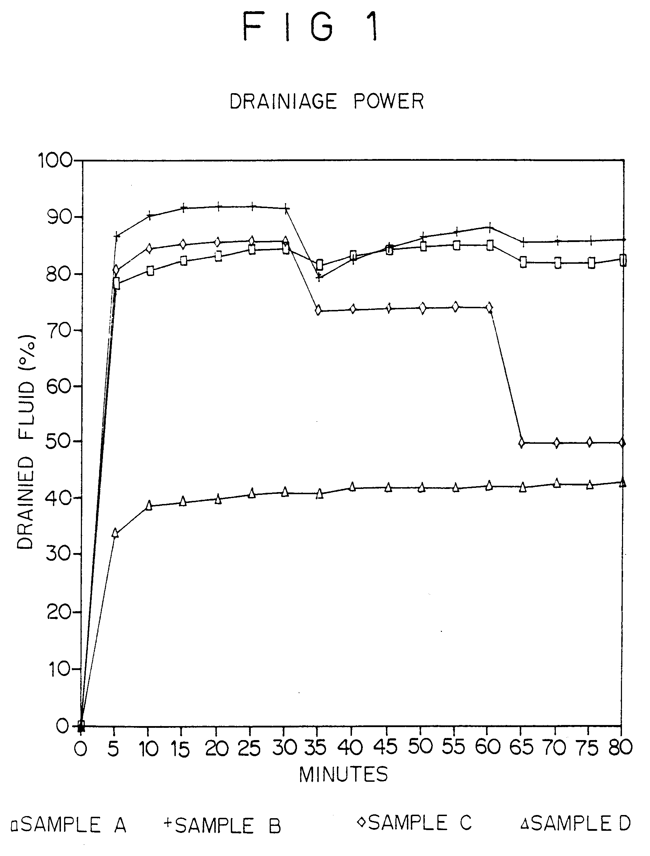

and then the results are placed in time graphs versus % drainage, as presented in figure 1.

**LONGITUDINAL SPREADING OF LIQUID**

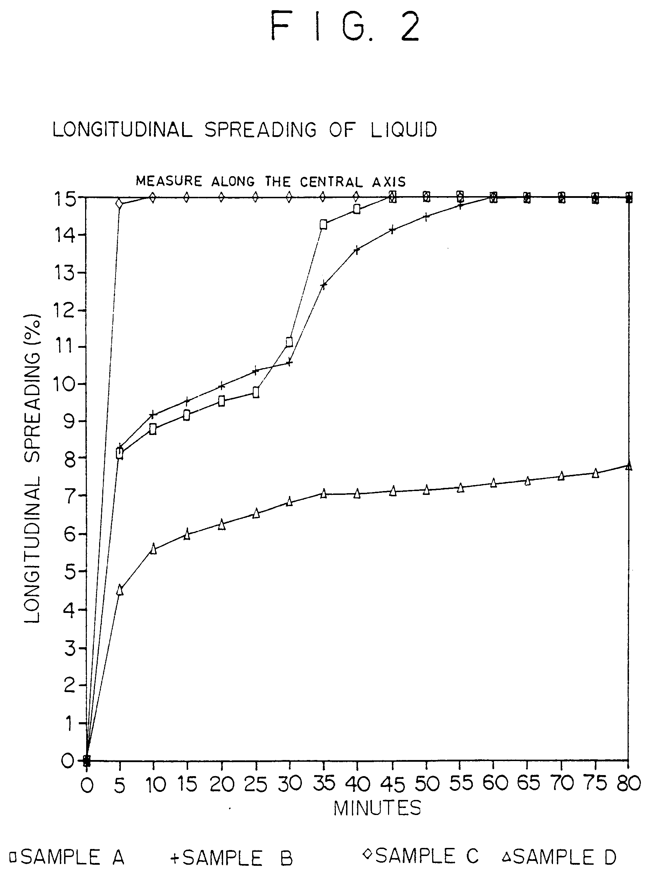

Measurements are made every 5 minutes with a scale graduated in cm, during 80 minutes, of the way gone by the drained fluid along the longitudinal central axis of the sample (of 5cm x 15cm) while the test for draining power is being carried out. The result is placed in a graph time versus longitudinal spreading (cm), as presented in figure 2 enclosed.

Table 1

| Sample | Absorbing capacity (g/g) | Holding capacity (%) | Thickness (cm) | Flexibility (appraisal) |
|--------|--------------------------|----------------------|----------------|--------------------------|
| A | 6.6 | 94.7 | 0.15 | adequate |
| B | 9.1 | 93.1 | 0.15 | adequate |
| C | 3.8 | 89.2 | 0.15 | adequate |
| D | 11.6 | 76.9 | 0.50 | excellent |
| Note: each measure is the mean of three runs. | | | | |

**ANALYSIS OF THE RESULTS**

The obtained results show the adequacy of the material used in the disposable absorbent pads of the invention, as compared with the prior art represented by woodpulp.

The measured parameters should not be analysed independently of one another, because the absorbing properties are interrelated.

Taking sample D, which represents the most usual absorbing medium in these articles, one can see that it has a high absorbing capacity and excellent flexibility. The other parameters, however, are inferior to all of the other samples.

With a higher density (sample C) cellulose pulp improves its behaviour in an exceptional manner as regards the longitudinal spreading and the draining power (in this aspect only in the beginning). The absorbing capacity decreases drastically and the holding capacity increases; it is necessary to take into account that the amount of liquid absorbed is quite less than in the other samples; the increased numeric value refers only to that little liquid. The flexibility also decreases.

Samples A and B, which relate to the pad of the invention comprising structured material, present a better holding capacity with respect to the woodpulp of Samples C and D. Its draining power is equivalent to that of those samples in the first 30 minutes, and then they become superior.

The longitudinal spreading of liquid after 60 minutes reach the same value as that obtained with Sample C, quite superior to that of Sample D.

As to the absorbing capacity, Sample A has a value adequate for absorbing smaller amounts of liquid. However, Sample B, which represents a simple treatment given to Sample A, presents a value near that of the cellulose pulp (Sample D), without having its shortcomings.

As regards the aspects of thickness and flexibility, Samples A and B according to the invention have adequate flexibility and low thickness, with advantages over Samples C and D as to the general absorbing performance.

These results show that the material of the invention has advantages over the prior art and is suitable for utilization in disposable absorbent articles such as diapers, women's hygienic pads or compresses, in a non-limitative manner.

**Claims**

1. An absorbent pad, comprising an absorbent material being produced from maize stem as an absorbent element.

2. The absorbent pad according to claim 1, characterized by containing the maize stem in granulated form.

3. The absorbent pad according to claim 1 or 2, characterized by having a granulometry of less than 10 mesh.

4. The absorbent pad according to any one of the preceding claims, characterized in that said stem is free from husk and nodes.

5. The absorbent pad according to any one of the preceding claims, characterized by being bleached.

6. The absorbent pad according to any one of the preceding claims, characterized in that the pad further comprises a binder or binders and/or fibers or filiform materials.

7. The absorbent pad according to any one of the preceding claims, characterized in that said pad comprises fibers chosen from polyester, polypropylene and rayon.

8. The absorbent pad according to any one of the preceding claims, characterized in that said fibers or filiform materials have an average length of from 1 to 25 mm.

9. The absorbent pad according to any one of the preceding claims, characterized in that said fibers of filiform materials have a tex (denier) between 0.011 and 2.22.(0.1 and 20).

10. The absorbent pad according to claim 7, characterized in that said polyester or polypropylene fiber portions comprise, in alternating layers, said absorbent materials.

11. The absorbent pad according to any one of the preceding claims comprising sheet(s) or foliate material.

12. The absorbent pad in accordance with claim 11, characterized in that the sheet(s) or foliate material is (are) chosen from paper, fabric, nonwoven fabric web or plastic.

13. The absorbent pad in accordance with claim 11 or 12 comprising sheets of paper having a weight from 10 to 50 g/m$^2$.

14. The absorbent pad in accordance with claim 11 or 13 comprising a layer of paper arranged on each of the surfaces of said pad.

15. The absorbent pad according to any one of the preceding claims, characterized in that its thickness is between 0.05 and 1 cm.

16. The absorbent pad according to any one of the preceding claims, characterized in that its apparent density is between 0.01 and 0.7 g/cm$^3$.

17. The absorbent pad according to any one of the preceding claims, characterized by comprising layers of fibers alternating with layers of said absorbent material, enclosed by a layer of paper on each surface of said pad, with a total thickness of about 0.15 cm and with an apparent density of 0.35 g/cm$^3$.

18. A method of obtaining the absorbent pad of claims 1 to 17 comprising the introduction of one or more structuring materials in the absorbent material.

19. The method of claim 18, characterized by further comprising a shaping step.

20. The method of claims 18 and 19, characterized in that the shaping operation is carried out by pressing.

21. The method of claims 18 to 20, characterized in that the introduction of structuring materials is carried out by contacting said absorbent material with a polymeric material in molten or almost molten state.

22. The method of claims 18 to 21, characterized in that the introduction of fibers, filiform materials, sheets or foliate materials, as structuring materials, is preceded by wetting of the absorbent material.

23. The method of claims 18 to 22, characterized by additionally comprising the treatment of said absorbent material against attack by microorganisms.

24. The method of claims 18 to 23, characterized by comprising a subsequent step of flexibilizing said absorbent pad.

25. The method of claim 24, characterized in that the subsequent flexibilization step is carried out by maceration with a gear-type calender.

26. A disposable absorbent article comprising at least one absorbent pad according to claims 1 - 18.

27. A disposable absorbent article in accordance with claim 26, characterized by being a disposable diaper, a woman's hygienic pad or a compress.

**Patentansprüche**

1. Absorbierendes Kissen, umfassend ein absorbierendes Material das aus Maisstengeln als einem absorbierenden Element hergestellt ist.

2. Absorbierendes Krissen nach Anspruch 1, dadurch gekennzeichnet, daß es die Maisstengel in granulierter Form enthält.

3. Absorbierendes Kissen nach Anspruch 1 oder 2, dadurch gekennzeichnet daß es eine Körnchengröße von weniger als 10 Mesh aufweist.

4. Absorhierendes Kissen nach einem der vorhergehenden Ansprüche, dadurch gekennzeichnet daß der Stengel frei von Hülsen und Knoten ist.

5. Absorbierendes Kissen nach einem der vorhergehenden Ansprüche dadurch gekennzeichnet, daß es gebleicht ist.

6. Absorbierendes Kissen nach einem der vorhergehenden Ansprüche dadurch gekennzeichnet, daß das Kissen darüber hinaus ein Bindemittel oder Bindemittel und/oder Fasern oder filiforme Materialien umfaßt.

7. Absorbierendes Kissen nach einem der vorhergehenden Ansprüche, dadurch gekennzeichnet daß das Kissen Fasern ausgewählt aus Polyester, Polypropylen und Rayon umfaßt.

8. Absorbierendes Kissen nach einem der vorhergehenden Ansprüche, dadurch gekennzeichnet, daß die Fasern oder filiformen Materialien eine durchschnittliche Länge von 1 bis 25 mm aufweisen.

9. Absorbierendes Kissen nach einem der vorhergehenden Ansprüche, dadurch gekennzeichnet, daß die Fasern der filiformen Materialien zwischen 0,011 und 2,22 tex (0,1 und 20 denier) aufweisen.

10. Absorbierendes Kissen nach Anspruch 7, dadurch gekennzeichnet, daß die Polyester- oder Polypropylenfaseranteile die absorbierenden Materialien in alternierenden Schichten umfassen.

11. Absorbierendes Kissen nach einem der vorhergehenden Ansprüche, das ein Blatt (Blätter) oder blattartiges Material umfaßt.

12. Absorbierendes Kissen nach Anspruch 11, dadurch gekennzeichnet, daß das Blatt (die Blätter) oder blattartige Material ausgewählt ist (sind) aus Papier, Stoff, Gewebe aus nichtgewebtem Stoff oder Kunststoff.

13. Absorbierendes Kissen nach Anspruch 11 oder 12, das Papierblätter mit einem Gewicht von 10 bis 50 g/m$^2$ umfaßt.

14. Absorbierendes Kissen nach Anspruch 11 oder 13, das eine Papierschicht umfaßt, die auf jeder der Oberflächen des Kissens angeordnet ist.

15. Absorbierendes Kissen nach einem der vorhergehenden Ansprüche, dadurch gekennzeichnet, daß seine Dicke zwischen 0,05 und 1 cm liegt.

16. Absorbierendes Kissen nach einem der vorhergehenden Ansprüche dadurch gekennzeichnet daß seine scheinbare Dichte zwischen 0,01 und 0,7 g/cm$^3$ beträgt.

17. Absorbierendes Kissen nach einem der vorhergehenden Ansprüche dadurch gekennzeichnet, daß es abwechselnd Faserschichten und Schichten des absorbierenden Materials umfaßt, die auf jeder Oberfläche des Kissens von einer Papierschicht umschlossen sind mit einer Gesamtdicke von etwa 0,15 cm und mit einer scheinbaren Dichte von 0,35 g/cm$^3$.

18. Verfahren zum Herstellen des absorbierenden Kissens der Ansprüche 1 bis 17, umfassend das Einführen von einem oder mehreren strukturgebenden Materialien in das absorbierende Material.

**19.** Verfahren nach Anspruch 18, darüber hinaus dadurch gekennzeichnet, daß es einen Formungsschritt umfaßt.

**20.** Verfahren nach Ansprüchen 18 und 19 dadurch gekennzeichnet, daß der Formungsvorgang durch Pressen durchgeführt wird.

**21.** Verfahren nach Ansprüchen 18 bis 20. dadurch gekennzeichnet , daß die Einführung von strukturgebenden Materialien durch Kontaktieren des absorbierenden Materials mit einem polymeren Material in geschmolzenem oder nahezu geschmolzenem Zustand durchgeführt wird.

**22.** Verfahren nach Ansprüchen 18 bis 21, dadurch gekennzeichnet daß der Einführung von Fasern, filiformen Materialien. Blättern oder blattartigen Materialien als strukturgebende Materialien, das Benetzen des absorbierenden Materials vorausgeht.

**23.** Verfahren nach Ansprüchen 18 bis 22, dadurch gekennzeichnet, daß es darüber hinaus das Behandeln des absorbierenden Materials gegen einen Angriff von Mikroorganismen umfaßt.

**24.** Verfahren nach Ansprüchen 18 bis 23, dadurch gekennzeichnet, daß es einen nachfolgenden Schritt der Flexibilisierung des absorbierenden Kissens umfaßt.

**25.** Verfahren nach Anspruch 24, dadurch gekennzeichnet, daß der nachfolgende Flexibilisierungsschritt durch Macerieren mit einem zahnradartigen Kalander durchgeführt wird.

**26.** Absorbierender Einwegartikel, umfassend mindestens ein absorbierendes Kissen nach Ansprüchen 1 bis 18.

**27.** Absorbierender Einwegartikel nach Anspruch 26, dadurch gekennzeichnet, daß er eine Einwegwindel, eine Damenbinde oder eine Kompresse ist.

## Revendications

**1.** Un tampon absorbant, comprenant en tant qu'élément absorbant une matière absorbante produite à partir de la tige du maïs.

**2.** Le tampon absorbant selon la Revendication 1, caractérisé en ce qu'il contient la tige de maïs sous forme granulée.

**3.** Le tampon absorbant selon la Revendication 1 ou 2, caractérisé en ce qu'il présente une granulométrie inférieure à 10 mesh.

**4.** Le tampon absorbant selon l'une quelconque des Revendications précédentes, caractérisé en ce que ladite tige est exempte d'enveloppe et de nodosités.

**5.** Le tampon absorbant selon l'une quelconque des Revendications précédentes, caractérisé en ce qu'il est blanchi.

**6.** Le tampon absorbant selon l'une quelconque des Revendications précédentes, caractérisé en ce que le tampon comprend en outre un liant ou des liants et/ou des fibres ou matières filiformes

**7.** Le tampon absorbant selon l'une quelconque des Revendications précédentes, caractérisé en ce que ledit tampon comprend des libres sélectionnées parmi du polyester, du polypropylène et de la rayonne.

**8.** Le tampon absorbant selon l'une quelconque des Revendications précédentes, caractérisé en ce que lesdites libres ou matières filiformes présentent une longueur moyenne comprise entre 1 et 25 mm

**9.** Le tampon absorbant selon l'une quelconque des Revendications précédentes, caractérisé en ce que lesdites fibres de matières filiformes présentent un tex (denier) compris entre 0,011 et 2,22(0,1 et 20).

**10.** Le tampon absorbant selon la Revendication 7, caractérisé en ce que lesdites portions de fibre de polyester ou de polypropylène comprennent, en couches alternées, lesdites matières absorbantes.

**11.** Le tampon absorbant selon l'une quelconque des Revendications précédentes, comprenant une ou des feuilles ou de la matière foliée.

**12.** Le tampon absorbant selon la Revendication 11, caractérisé en ce que la ou les feuille(s) ou la matière foliée est (sont) sélectionnée(s) parmi du papier, du tissu, un tissu non tissé ou du plastique.

**13.** Le tampon absorbant selon la Revendication 11 ou 12, comprenant des feuilles de papier présentant une masse comprise entre 10 et 50 g/m$^2$.

**14.** Le tampon absorbant selon la Revendication 11 ou 13, comprenant une couche de papier disposée sur chacune des surfaces du dit tampon.

**15.** Le tampon absorbant selon l'une quelconque des Revendications précédentes, caractérisé en ce que son épaisseur est comprise entre 0,05 et 1 cm.

**16.** Le tampon absorbant selon l'une quelconque des Revendications précédentes, caractérisé en ce que sa densité apparente est comprise entre 0,01 et 0,7g/cm$^3$.

**17.** Le tampon absorbant selon l'une quelconque des Revendications précédentes, caractérisé en ce qu'il comprend des couches de fibres en alternance avec des couches de ladite matière absorbante, enfermées par une couche de papier sur chaque surface du dit tampon, et présentant une épaisseur totale environ égale à 0,15 cm et une densité apparente égale à 0,35 g/cm$^3$.

**18.** Une méthode de fabrication du tampon absorbant selon les Revendications 1 à 17, comprenant l'introduction d'une ou de plusieurs matières structurantes dans la matière absorbante.

**19.** La méthode selon la Revendication 18, caractérisée en ce qu'elle comprend en outre une étape de façonnage.

**20.** La méthode selon les Revendications 18 et 19, caractérisée en ce que l'opération de façonnage est effectuée par compression.

**21.** La méthode selon les Revendications 18 à 20, caractérisée en ce que l'introduction de matières structurantes est effectuée en mettant en contact ladite matière absorbante avec une matière polymère à l'état fondu ou presque fondu.

**22.** La méthode selon les Revendications 18 à 21, caractérisée en ce que l'introduction de fibres, de matières filiformes, de feuilles ou matières foliées, en tant que matières structurantes, est précédée par le mouillage de la matière absorbante.

**23.** La méthode selon les Revendications 18 à 22, caractérisée en ce qu'elle comprend en outre le traitement de ladite matière absorbante contre une attaque par des micro-organismes.

**24.** La méthode selon les Revendications 18 à 23, caractérisée en ce qu'elle comprend une étape ultérieure de flexibilisation du dit tampon absorbant.

**25.** La méthode selon la Revendication 24, caractérisée en ce que l'étape ultérieure de flexibilisation est effectuée par macération avec une calandre de type dentée.

**26.** Un article absorbant jetable comprenant au moins un tampon absorbant en accord avec les Revendications 1 à 18.

**27.** Un article absorbant jetable en accord avec la Revendication 26, caractérisé en ce qu'il est une couche jetable, une serviette hygiénique pour femmes ou une compresse.

# F I G 1

DRAINIAGE POWER

□SAMPLE A    +SAMPLE B    ◇SAMPLE C    △SAMPLE D

# F I G. 2

LONGITUDINAL SPREADING OF LIQUID

MEASURE ALONG THE CENTRAL AXIS

□SAMPLE A    +SAMPLE B    ◇SAMPLE C  △SAMPLE D